# EUROPEAN PATENT APPLICATION

(11) **EP 3 978 018 A1**
(43) Date of publication of application: **06.04.2022**
(21) Application number: 20813246.4
(22) Date of filing: 29.05.2020
(51) Int. Cl.: A61K 45/00, A61K 48/00, A61P 1/18, A61P 35/00, A61P 43/00, A61K 38/12, G01N 33/15, G01N 33/50, A61K 31/7088, A61K 31/7105, A61K 31/713

(54) **NOVEL THERAPEUTIC AGENT FOR DIGESTIVE ORGAN CANCER, AND SCREENING METHOD FOR SAME**

(30) Priority: 31.05.2019 JP 2019103384; 07.04.2020 JP 2020069417
(71) Applicant: Osaka University, Suita-shi, Osaka 565-0871 (JP)
(72) Inventor: KISHIMOTO, Tadamitsu, Suita-shi, Osaka 565-0871 (JP); HASHIMOTO, Shigeru, Suita-shi, Osaka 565-0871 (JP)
(74) Representative: Witte, Weller & Partner Patentanwälte mbB
(86) International application number: PCT/JP2020/021278
(87) International publication number: WO 2020/241816

(57) **Abstract**

The present invention provides a therapeutic agent for gastrointestinal cancer, comprising an Arid5A inhibitor as an active ingredient; and a method for screening for a candidate substance useful for treating gastrointestinal cancer, comprising the steps of: (1) examining whether a test substance affects Arid5A expression, and (2) identifying the test substance as screen positive when the test substance is capable of reducing Arid5A expression based on comparison of cases with and without the test substance.

## Description

### TECHNICAL FIELD

The present invention relates to a novel therapeutic agent for gastrointestinal cancer and a method for screening for the same.

### BACKGROUND ART

Pancreatic cancer is very difficult to detect early, and in many cases, local progression or distant metastasis is observed at the time of diagnosis, leading to a very poor prognosis. Current treatments include surgical resection, radiation therapy, and anticancer drugs such as gemcitabine, but all have limited efficacy. Pancreatic cancer often originates from the pancreatic ductal epithelium, and a subgroup with an epithelial-to-mesenchymal transition (EMT) gene profile has been shown to have a poor prognosis.

Furthermore, previous reports have shown that angiogenesis is suppressed due to increased stromal reaction (fibrosis) in tumor tissues, resulting in hypotrophic and hypoxic conditions and less infiltration of cytotoxic T cells, CD4+ Th1 helper T cells, natural killer cells, etc. Therefore, immune checkpoint inhibitory antibodies, which have attracted much attention in recent years, are less effective in treating pancreatic cancer. CAR-T therapies targeting MUC1 or mesothelin have also been attempted, but no significant antitumor effect has been demonstrated.

On the other hand, elevated blood IL-6 levels have been shown to correlate with poor prognosis in pancreatic cancer patients. In addition, previous reports have shown that IL-6 signaling is involved in the development and progression of pancreatic cancer based on the analysis of pancreatic cancer mouse models etc. Therefore, it is highly desired to create diagnostic and therapeutic methods for pancreatic cancer via targeting molecules involved in IL-6 signaling or IL-6 production enhancement, but such methods have not yet been developed.

AT Rich Interactive Domain 5A (hereinafter abbreviated as Arid5A) has been reported as an IL-6 mRNA stabilizing protein (see Non-Patent Literature 1). The present inventors have found that Arid5A inhibitors are effective in treating sepsis and pulmonary diseases (Patent literature 1 and 2). In addition, Arid5A gene is known to be differentially expressed in several types of cancers, suggesting that Arid5A gene may be one of the candidate genes for detecting or diagnosing cancer (Patent literature 3 to 6). In prostate cancer cell lines, it is known that knocking down Arid5A gene expression suppresses cell growth (Non-Patent Literature 2).

### CITATION LIST

### Patent Literature

Patent Literature 1: WO 2016/104490
Patent Literature 2: WO 2017/002928
Patent Literature 3: WO 2008/087040
Patent Literature 4: WO 2014/187959
Patent Literature 5: WO 2015/049289
Patent Literature 6: WO 2017/083513

### Non-Patent Literature

Non-Patent Literature 1: PNAS, 110, 23, 9404-9414 (2013)
Non-Patent Literature 2: Cancer Res, 77 (13 Suppl), Abstract 4489 (2017)

### SUMMARY OF INVENTION

### TECHNICAL PROBLEM

An object of the present invention is to provide a novel therapeutic agent for gastrointestinal cancer and a method for screening for a candidate substance useful for treating gastrointestinal cancer.

### SOLUTION TO PROBLEM

The present invention includes the following to achieve the above-mentioned object.
[1] A therapeutic agent for gastrointestinal cancer, comprising an Arid5A inhibitor as an active ingredient.
[2] The therapeutic agent according to the above [1], wherein the Arid5A inhibitor comprises a substance capable of reducing Arid5A expression or function.
[3] The therapeutic agent according to the above [1] or [2], wherein the Arid5A inhibitor is a nucleic acid oligomer or a polypeptide.
[4] The therapeutic agent according to the above [3], wherein the nucleic acid oligomer is selected from the group consisting of an siRNA, an shRNA, an antisense nucleic acid, a decoy nucleic acid, a nucleic acid aptamer, and a ribozyme.
[5] The therapeutic agent according to the above [3], wherein the polypeptide is a cyclic polypeptide.
[6] The therapeutic agent according to any one of the above [1] to [5], wherein the Arid5A inhibitor is capable of inhibiting epithelial-to-mesenchymal transition of cancer cells.
[7] The therapeutic agent according to any one of the above [1] to [6], wherein the Arid5A inhibitor is capable of inhibiting cancer cell invasion.
[8] The therapeutic agent according to any one of the above [1] to [7], wherein the gastrointestinal cancer is pancreatic cancer or colorectal cancer.
[9] A method for screening for a candidate substance useful for treating gastrointestinal cancer, comprising the steps of:
   (1) examining whether a test substance affects Arid5A expression, and
   (2) identifying the test substance as screen positive when the test substance is capable of reducing Arid5A expression based on comparison of cases with and without the test substance.
[10] A method for screening for a candidate substance useful for treating gastrointestinal cancer, comprising the steps of:
   (a) examining whether a test substance affects Arid5A function, and
   (b) identifying the test substance as screen positive when the test substance is capable of reducing Arid5A function based on comparison of cases with and without the test substance.
[11] The screening method according to the above [10], wherein the Arid5A function is stabilization of any one mRNA selected from the group consisting of IL-6 mRNA, IDO1 mRNA, CXCL3 mRNA, CCL2 mRNA, CCL5 mRNA, CCL7 mRNA, and CCL8 mRNA.
[12] The screening method according to any one of the above [9] to [11], wherein the gastrointestinal cancer is pancreatic cancer or colorectal cancer.

The present invention further includes the following.
[101] A therapeutic agent for pancreatic cancer, comprising an Arid5A inhibitor as an active ingredient.
[102] The therapeutic agent for pancreatic cancer according to the above [101], wherein the Arid5A inhibitor is a nucleic acid oligomer or a polypeptide.
[103] The therapeutic agent for pancreatic cancer according to the above [102], wherein the nucleic acid oligomer is selected from the group consisting of an siRNA, an shRNA, an antisense nucleic acid, a decoy nucleic acid, a nucleic acid aptamer, and a ribozyme.
[104] The therapeutic agent for pancreatic cancer according to the above [102], wherein the polypeptide is a cyclic polypeptide.
[105] The therapeutic agent according to any one of the above [101] to [104], wherein the Arid5A inhibitor is capable of inhibiting epithelial-to-mesenchymal transition of cancer cells.
[106] The therapeutic agent according to any one of the above [101] to [105], wherein the Arid5A inhibitor is capable of inhibiting cancer cell invasion.
[107] A method for screening for a candidate substance useful for treating pancreatic cancer, comprising the steps of:
   (1) examining whether a test substance affects Arid5A expression, and
   (2) identifying the test substance as screen positive when the test substance is capable of reducing Arid5A expression based on comparison of cases with and without the test substance.
[108] A method for screening for a candidate substance useful for treating pancreatic cancer, comprising the steps of:
   (a) examining whether a test substance affects Arid5A function, and
   (b) identifying the test substance as screen positive when the test substance is capable of reducing Arid5A function based on comparison of cases with and without the test substance.
[109] The screening method according to the above [108], wherein the Arid5A function is stabilization of IL-6 mRNA or stabilization of IDO1 mRNA.

### ADVANTAGEOUS EFFECTS OF INVENTION

The present invention provides a novel therapeutic agent for gastrointestinal cancer. The present invention also provides a novel method for screening for a candidate substance useful for treating gastrointestinal cancer.

### BRIEF DESCRIPTION OF DRAWINGS

Fig. 1 shows the results of western blot analysis for Arid5a expression in normal mouse embryonic fibroblasts (MEF), metastatic mouse melanoma cells (B16/F10) , and human pancreatic ductal adenocarcinoma model mouse (KPC mouse)-derived cells (KPC) .
Fig. 2 shows the deletion regions in exon 2 and exon 5 of Arid5a used in generating Arid5a-deficient KPC cells using the CRISPR/Cas9 system.
Fig. 3 shows the results of PCR-based verification of the deletions of exon 2 and exon 5 of the Arid5a gene in the generated Arid5a-deficient KPC cells (KO4 and KO5).
Fig. 4 shows the results of qRT-PCR analysis for Arid5a mRNA in Arid5a-deficient (Arid5a-/-) KPC cells and wild-type (WT) KPC cells.
Fig. 5 shows the results of western blot analysis for Arid5a protein in Arid5a-deficient KPC cells and wild-type KPC cells.
Fig. 6 shows the tumor volume measured over time after subcutaneous administration of Arid5a-deficient KPC cells and wild-type KPC cells to the left and right flanks of 5- to 7-week-old female C57BL/6 mice.
Fig. 7 shows the tumor volume measured over time after subcutaneous administration of Arid5a-deficient KPC cells and wild-type KPC cells to the left and right flanks of 5- to 7-week-old female BALB/c nude mice.
Fig. 8 shows the results of microscopic analysis of cell morphology of Arid5a-deficient KPC cells and wild-type KPC cells cultured in the presence or absence of IL-6 for 48 hours.
Fig. 9 shows the results of microscopic analysis of cell morphology of Arid5a-deficient KPC cells and wild-type KPC cells cultured in the presence or absence of TGFβ for 48 hours.
Fig. 10 shows the results of western blot analysis for E-cadherin expression in Arid5a-deficient KPC cells and wild-type KPC cells cultured in the presence or absence of IL-6 or TGFβ for 48 hours.
Fig. 11 is a schematic view of IL-6-induced Matrigel invasion assay.
Fig. 12 shows the results of IL-6-induced Matrigel invasion assay for Arid5a-deficient KPC cells, Arid5a-deficient KPC cells transfected with an Arid5a expression vector, and wild-type KPC cells.
Fig. 13 shows the cell growth of Arid5a-deficient KPC cells, Arid5a-deficient KPC cells transfected with an Arid5a expression vector, and wild-type KPC cells.
Fig. 14 shows the results of RNA sequencing of Arid5a-deficient KPC cells and wild-type KPC cells for evaluating the changes in the expressions of Idol, Ido2, and Tod2 as a result of Arid5a deletion.
Fig. 15 shows the results of the Cancer Genome Atlas (TCGA) dataset analysis of the correlation between IDO1 expression and ARID5a expression in primary pancreatic ductal adenocarcinoma (PDAC) patients.
Fig. 16 shows the results of the Cancer Genome Atlas (TCGA) dataset analysis of survival probability in a group of primary pancreatic ductal adenocarcinoma (PDAC) patients with high expression of IDO1 mRNA and in a group of primary PDAC patients with low expression of IDO1 mRNA.
Fig. 17 shows the results of qRT-PCR analysis for Idol mRNA expression in Arid5a-deficient KPC cells and wild-type KPC cells cultured in the presence or absence of IFN-γ for 48 hours.
Fig. 18 shows the results of western blot analysis for Arid5a protein and Idol protein in Arid5a-deficient KPC cells and wild-type KPC cells cultured in the presence or absence of IFN-γ for 24, 48, and 72 hours.
Fig. 19 shows the results of western blot analysis for Arid5a protein and Idol protein in Arid5a-deficient KPC cells transfected with an Arid5a expression vector and Arid5a-deficient KPC cells transfected with an empty vector after 48 hours of culture in the presence or absence of IFN-γ.
Fig. 20 shows the results of ELISA for measuring kynurenine levels in the supernatants of Arid5a-deficient KPC cells and wild-type KPC cells cultured in the presence or absence of IFN-γ for 24, 48, and 72 hours.
Fig. 21 shows the results of 3'-UTR luciferase assay in Arid5a-deficient KPC cells transfected with an Arid5a pcDNA3-Flag-overexpressing vector or an empty pcDNA3-Flag vector.
Fig. 22 shows the expression levels of Arid5a mRNA measured in human glioblastoma cell lines A172 and T98G transfected with Arid5a siRNA.
Fig. 23 shows the expression levels of Idol mRNA measured in human glioblastoma cell lines A172 and T98G transfected with Arid5a siRNA.
Fig. 24 shows the results of western blot analysis for Stat1 and pStat1 in Arid5a-deficient KPC cells and wild-type KPC cells cultured in the presence or absence of IFN-γ for 10 minutes, and 1, 6, 12, 24, 48, and 72 hours.
Fig. 25 shows the results of western blot analysis for Arid5a and Idol after IFN-γ treatment of Stat1-silenced KPC cells (#1 and #5), which were generated by lentiviral transduction of KPC cells with Stat1 shRNA, and of control cells (Scr), which were generated by lentiviral transduction of KPC cells with scrambled shRNA.
Fig. 26 shows the results of RNA sequencing of Arid5a-deficient KPC cells and wild-type KPC cells for evaluating the changes in the expressions of various chemokines as a result of Arid5a deletion.
Fig. 27 shows the results of the Cancer Genome Atlas (TCGA) dataset analysis of the correlation between chemokine (CXCL3, CCL2, CCL7, and CCL8) expression and ARID5a expression in primary pancreatic ductal adenocarcinoma (PDAC) patients.
Fig. 28 shows the results of qRT-PCR analysis for mRNA expression levels of chemokines (CXCL3, CCL2, CCL7, and CCL8) in Arid5a-deficient KPC cells and wild-type KPC cells cultured for 48 hours.
Fig. 29 shows the results of 3'-UTR luciferase assay in Arid5a-deficient KPC cells transfected with an Arid5a pcDNA3-Flag-overexpressing vector or an empty pcDNA3-Flag vector. The luciferase activity was normalized to Renilla luciferase activity as an internal control and then further normalized to the luciferase activity measured in the empty vector-transfected cells.
Fig. 30 shows the tumor volume measured over time after subcutaneous administration of wild-type MC38 cells and Arid5a-deficient MC38 cells to the left and right flanks of 5-to 7-week-old female C57BL/6 mice. Data obtained after subcutaneous administration of wild-type KPC cells or Arid5a-deficient KPC cells as in Fig. 6 are also shown in this figure.
Fig. 31 shows the tumor volume measured over time after subcutaneous administration of wild-type MC38 cells and Arid5a-deficient MC38 cells to the left and right flanks of 5-to 7-week-old female BALB/c nude mice.
Fig. 32 shows the results of the Cancer Genome Atlas (TCGA) dataset analysis of survival probability in a group of colorectal cancer patients with high expression of Arid5a mRNA and in a group of colorectal cancer patients with low expression of Arid5a mRNA.

### DESCRIPTION OF EMBODIMENTS

### Therapeutic agent for gastrointestinal cancer

The present invention provides a therapeutic agent for gastrointestinal cancer comprising an Arid5A inhibitor as an active ingredient. The present inventors found that Arid5A is involved in immune evasion of pancreatic and colorectal cancer cells and demonstrated that Arid5A inhibition results in suppressed growth of pancreatic and colorectal cancers. Therefore, Arid5A inhibitors may be useful for treating gastrointestinal cancer. In one embodiment of the present invention, the gastrointestinal cancer represents, for example, esophageal cancer, stomach cancer, liver cancer, biliary tract cancer, pancreatic cancer, duodenal cancer, small intestinal cancer, colorectal cancer, etc. In certain embodiments, the gastrointestinal cancer represents pancreatic cancer or colorectal cancer. In the present invention, the pancreatic cancer may be pancreatic ductal adenocarcinoma (PDAC). The present invention also provides a method for treating gastrointestinal cancer comprising administering an Arid5A inhibitor, use of an Arid5A inhibitor in the production of a therapeutic agent for gastrointestinal cancer, and an Arid5A inhibitor for use in the treatment of gastrointestinal cancer.

The "Arid5A" in the present invention refers to AT Rich Interactive Domain 5A, which has been reported as an IL-6 mRNA stabilizing protein. For example, human Arid5A includes various isoforms (NCBI accession numbers NP997646.1, XP006712266.1, XP006712265.1, XP005263918.1, XP005263913.1, XP005263917.1, etc.). Similarly, mouse Arid5A includes various isoforms (NCBI accession numbers NP00165676.1, NP00165677.1, NP001277655.1, NP001277656.1, NP666108.2, etc.).

The term "Arid5A inhibitor" in the present invention refers to a substance capable of inhibiting Arid5A expression and/or function. The Arid5A inhibitor may be a substance capable of directly inhibiting the expression of Arid5A or a substance capable of inhibiting the biological function of Arid5A by binding to Arid5A. Alternatively, the Arid5A inhibitor may be a substance capable of indirectly inhibiting the biological function of Arid5A by binding to a molecule affected by Arid5A (IL-6 mRNA, IDO1 mRNA, CXCL3 mRNA, CCL2 mRNA, CCL5 mRNA, CCL7 mRNA, CCL8 mRNA, or the like) . Since the inhibition of Arid5A expression results in the inhibition of Arid5A function as well, the former can be considered as one of the embodiments included in the latter. As used herein, the wording "inhibiting Arid5A expression and/or function" can be rephrased as "reducing Arid5A expression and/or function".

The Arid5A inhibitor may be a substance capable of inhibiting IL-6 mRNA stabilization by binding to IL-6 mRNA competitively with Arid5A. For example, human IL-6 mRNA includes various isoforms (NCBI accession numbers NM_000600.3, XM_005249745.2, etc.). Similarly, mouse IL-6 mRNA includes various isoforms (NCBI accession number NM_031168.1, etc.). The Arid5A inhibitor may be a substance capable of inhibiting IDO1 mRNA stabilization by binding to IDO1 mRNA competitively with Arid5A. For example, human IDO1 mRNA includes various isoforms (NCBI accession numbers NM_002164.5, AK313259.1, etc.). Similarly, mouse IDO1 mRNA includes various isoforms (NCBI accession numbers NM_001293690.1, NM_008324.2, etc.).

The Arid5A inhibitor may be a substance capable of inhibiting CXCL3 mRNA stabilization by binding to CXCL3 mRNA competitively with Arid5A. For example, human CXCL3 mRNA includes various isoforms (NCBI accession number NM_002090.3, etc.). Similarly, mouse CXCL3 mRNA includes various isoforms (NCBI accession number NM_203320.3, etc.). The Arid5A inhibitor may be a substance capable of inhibiting CCL2 mRNA stabilization by binding to CCL2 mRNA competitively with Arid5A. For example, human CCL2 mRNA includes various isoforms (NCBI accession number NM_002982.4, etc.). Similarly, mouse CCL2 mRNA includes various isoforms (NCBI accession number NM_011333.3, etc.).

The Arid5A inhibitor may be a substance capable of inhibiting CCL7 mRNA stabilization by binding to CCL7 mRNA competitively with Arid5A. For example, human CCL7 mRNA includes various isoforms (NCBI accession number NM_006273.4, etc.). Similarly, mouse CCL7 mRNA includes various isoforms (NCBI accession number NM_013654.3, etc.). The Arid5A inhibitor may be a substance capable of inhibiting CCL8 mRNA stabilization by binding to CCL8 mRNA competitively with Arid5A. For example, human CCL8 mRNA includes various isoforms (NCBI accession number NM_005623.3, etc.). Similarly, mouse CCL8 mRNA includes various isoforms (NCBI accession number NM_021443.3, etc.).

The Arid5A inhibitor may be a substance capable of inhibiting CCL5 mRNA stabilization by binding to CCL5 mRNA competitively with Arid5A. For example, human CCL5 mRNA includes various isoforms (NCBI accession numbers NM_001278736.2, NM_002985.3, etc.). Similarly, mouse CCL5 mRNA includes various isoforms (NCBI accession number NM_013653.3, etc.).

The Arid5A inhibitor may be a substance capable of inhibiting the stabilization of any one mRNA selected from the group consisting of IL-6 mRNA, IDO1 mRNA, CXCL3 mRNA, CCL2 mRNA, CCL5 mRNA, CCL7 mRNA, and CCL8 mRNA or a substance capable of inhibiting the stabilization of two or more mRNAs selected from the same group.

Examples of the Arid5A inhibitor in the present invention include, but are not limited to, proteins such as anti-Arid5A antibodies, polypeptides, nucleic acid oligomers, and low molecular weight compounds such as chlorpromazine. A preferable example of the Arid5A inhibitor of the present invention is a protein, more preferably an antibody (anti-Arid5A antibody). Another preferable example of the Arid5A inhibitor of the present invention is a nucleic acid oligomer, more preferably an siRNA. Another preferable example of the Arid5A inhibitor of the present invention is a polypeptide, more preferably a cyclic polypeptide. An exemplary low molecular weight compound that is an Arid5A inhibitor is, for example, chlorpromazine (PNAS, 110, 23, 9409-9414 (2013)).

The inhibition of Arid5A expression can be achieved, for example, by using RNA interference effects on Arid5A gene expression. RNA interference is a method for suppressing gene expression by using RNAs (Genes and Development, 16, 948-958 (2002)). A double-stranded RNA having an identical or similar sequence to a target gene is introduced into a cell, resulting in suppressed expression of both the introduced foreign gene and the endogenous target gene. More specifically, siRNAs or antisense nucleic acids that exhibit RNA interference effects on Arid5A gene expression can be used to inhibit Arid5A gene expression.

The "nucleic acid oligomer" in the present invention refers to a nucleic acid oligomer capable of controlling the function of the Arid5A gene or protein. The nucleic acid oligomer may be an oligomer based on a natural or unnatural RNA or DNA. The nucleic acid oligomer in the present invention includes siRNAs, shRNAs, antisense nucleic acids, decoy nucleic acids, nucleic acid aptamers, and ribozymes.

The nucleic acid oligomer in the present invention is preferably an siRNA or an shRNA. The siRNA refers to a double-stranded RNA consisting of short strands without exhibiting cytotoxicity and can be, for example, 15 to 49 base pairs, preferably 15 to 35 base pairs, and more preferably 21 to 30 base pairs in length. The shRNA is a double-stranded RNA formed via a hairpin loop in a single-stranded RNA.

The siRNA and the shRNA do not have to be completely identical to the target gene, but have at least 70% or more, preferably 80% or more, more preferably 90% or more, and most preferably 95% or more sequence homology to the target gene.

In the double-stranded RNA portion of the siRNA and the shRNA, RNA strands are paired to each other, but may be not completely paired and may be partly unpaired due to a mismatch (a corresponding base is not complementary), a bulge (no corresponding base on the other strand), etc. In the present invention, the double-stranded RNA portion of the siRNA and the shRNA in which RNA strands are paired to each other may contain both a bulge and a mismatch.

The "antisense nucleic acid" in the present invention refers to an antisense nucleic acid that is complementary to the transcript of an Arid5A-encoding DNA. There are a number of causes for the action of antisense nucleic acids in suppressing target gene expression, including: RNase-mediated degradation triggered by RNA duplex recognition; inhibition of transcription initiation by triplex formation; transcription inhibition by hybrid formation at a site with a local open loop structure generated by an RNA polymerase; transcription inhibition by hybrid formation with the RNA being synthesized; splicing inhibition by hybrid formation at an intron-exon junction; splicing inhibition by hybrid formation at the site of spliceosome formation; inhibition of transport from the nucleus to the cytoplasm by hybrid formation with mRNA; inhibition of translation by hybrid formation at the translation initiation factor binding site; inhibition of peptide chain elongation by hybrid formation in the translational region of mRNA or at the polysome binding site of mRNA; and inhibition of gene expression by hybrid formation at the protein-nucleic acid interaction sites. In particular, the antisense nucleic acid suppresses the expression of the target gene by inhibiting the process of transcription, splicing, or translation.

The antisense sequence used in the present invention may suppress the expression of the target gene based on any of the actions described above. In one embodiment, designing an antisense sequence complementary to the untranslated region near the 5' end of Arid5A mRNA would be effective in inhibiting gene translation. Alternatively, a sequence complementary to the coding region or the 3' untranslated region can also be used. Thus, sequences complementary to the untranslated region as well as the translated region of the gene can be used. Therefore, nucleic acids containing an antisense sequence not only for the translated region of the gene but also for the untranslated region of the gene are included in the antisense nucleic acid used in the present invention. The antisense nucleic acid preferably has 90% or more, most preferably 95% or more complementarity to the transcript of the target gene. The length of the antisense RNA is not particularly limited as long as it enables effective inhibition of the expression of the target gene with the use of the antisense sequence.

In one embodiment, the "decoy nucleic acid" in the present invention is a nucleic acid oligomer that is homologous to an IL-6 mRNA sequence recognized by Arid5A. The decoy nucleic acid binds to Arid5A in place of the IL-6 mRNA sequence and inhibits Arid5A function. In another embodiment, the "decoy nucleic acid" in the present invention is a nucleic acid oligomer that is homologous to an IDO1 mRNA sequence recognized by Arid5A. The decoy nucleic acid binds to Arid5A in place of the IDO1 mRNA sequence and inhibits Arid5A function. In another embodiment, the "decoy nucleic acid" in the present invention is a nucleic acid oligomer that is homologous to a CXCL3 mRNA sequence, a CCL2 mRNA sequence, a CCL5 mRNA sequence, a CCL7 mRNA sequence, or a CCL8 mRNA sequence recognized by Arid5A. The decoy nucleic acid binds to Arid5A in place of the mRNA sequence described above and inhibits Arid5A function.

The ribozyme in the present invention refers, for example, to a molecule that is capable of cleaving Arid5A mRNA and inhibiting the translation of Arid5A protein.

The ribozyme can be designed from a gene sequence encoding Arid5A protein. For example, a hammerhead ribozyme can be produced using the method described in FEBS Letter, Vol. 228, pp. 228-230 (1988) . Not only a hammerhead ribozyme, but also a hairpin ribozyme, a delta ribozyme, and any other type of ribozyme may be included in the ribozyme herein as long as it is capable of cleaving Arid5A mRNA and inhibiting the translation of Arid5A protein.

The "cyclic polypeptide" in the present invention refers to a polypeptide that contains a cyclic structure formed of 4 or more amino acids and/or amino acid analogues. The cyclic polypeptide may have a linear portion in addition to the cyclic portion. The mode of linkage in the cyclic portion is not particularly limited and may be other than an amide or ester bond. Preferable examples of the mode of linkage in the cyclic portion include covalent bonds such as an amide bond, a carbon-carbon bond, a disulfide bond, an ester bond, a thioester bond, a thioether bond, a lactam bond, a bond mediated by an azoline skeleton, a bond mediated by a triazole structure, and a bond mediated by a fluorophore structure. The functional group used for cyclization, such as a carboxy group or an amino group, may be located on the main chain or on the side chain, as long as it allows cyclization. The "mode of linkage in the cyclic portion" herein refers to a mode of linkage in the portion where a ring is formed by cyclization. Examples of the amino acid analogue herein include hydroxycarboxylic acids (hydroxy acids).

Without any intention to limit the scope of the present invention, in some embodiments, the Arid5A inhibitor in the present invention may be a polypeptide, and the polypeptide may be a cyclic polypeptide. In some embodiments, the molecular weight of the cyclic polypeptide in the present invention may be 500 to 4000, 500 to 3000, or 500 to 2000.

In some embodiments, the cyclic polypeptide in the present invention may contain at least one kind of component selected from the group consisting of natural amino acids, unnatural amino acids, and amino acid analogues . The ratio of these amino acid components is not particularly limited.

In some embodiments, the total number of amino acids and amino acid analogues in the cyclic polypeptide of the present invention may be 4 to 20, 4 to 15, 6 to 15, 8 to 15, 9 to 13, or 10 to 13. In certain embodiments, the total number of amino acids and amino acid analogues in the cyclic portion of the cyclic polypeptide in the present invention may be 5 to 15, 7 to 12, or 9 to 11.

In some embodiments, when the cyclic polypeptide in the present invention has a linear portion, the number of amino acids and/or amino acid analogues in the linear portion is preferably 0 to 8, more preferably 0 to 5, and still more preferably 0 to 3. In a non-limiting embodiment, the "linear portion" herein may contain a natural amino acid, a non-natural amino acid (including a chemically modified or skeleton-converted amino acid), and/or an amino acid analogue.

In some embodiments, the polypeptide in the present invention may have a modification for improved cellular internalization. Such a modification is not particularly limited and can be achieved by known methods. For example, attachment of a cell membrane-permeable peptide can be used for such a modification. The cell membrane-permeable peptide can be a cell membrane-permeable peptide having a known sequence, such as a Tat peptide derived from the HIV Tat protein (Brooks, H. et al., Advanced Drug Delivery Reviews, Vol 57, Issue 4,2005, p.559-577), or a polyarginine consisting of 6 to 12 arginine residues (Nakase, I. et al., Advanced Drug Delivery Reviews, Vol 60, 2008, p.598-607). Attachment of a fatty acid or a stilbene derivative also reportedly allows peptides to access the cytoplasm (Covic, L. et al. (2002) Nat Med. 8:1161; Endres, P. J. et al. (2006) Molecular Imaging 4: 485; and Goubaeva, F. et al., J. Biol. Chem. 278:19634). These methods can be used to facilitate cellular internalization of the polypeptide.

For the production of the cyclic polypeptide of the present invention, for example, a cyclic polypeptide library may be used to identify a cyclic polypeptide capable of binding to Arid5A. Such an identification method is known, for example, in WO 2013/100132, WO 2012/033154, etc. Alternatively, the cyclic polypeptide may be produced by a chemical synthesis method, such as a liquid-phase synthesis method, a solid-phase synthesis method using Fmoc or Boc, or a combination of these methods.

The therapeutic agent for gastrointestinal cancer may further comprise an ingredient that is acceptable for use in the formulation, such as a preservative or a stabilizer. The term "acceptable for use in the formulation" means that an ingredient of interest is administrable with the therapeutic agent for gastrointestinal cancer, which itself may or may not have therapeutic effect on gastrointestinal cancer. Alternatively, the ingredient that is acceptable for use in the formulation may be an ingredient that has no therapeutic effect but has synergistic effect or additive stabilizing effect when used in combination with the Arid5A inhibitor.

Examples of the ingredient that is acceptable for use in the formulation include sterile water, physiological saline, stabilizers, excipients, buffers, preservatives, surfactants, chelating agents (such as EDTA), and binders.

Examples of the surfactant used in the present invention include nonionic surfactants, and typical examples include sorbitan fatty acid esters such as sorbitan monocaprylate, sorbitan monolaurate, and sorbitan monopalmitate; and glycerin fatty acid esters such as glycerin monocaprylate, glycerin monomyristate, and glycerin monostearate, with the proviso that these have an HLB of 6 to 18.

Anionic surfactants can also be included in the scope of the surfactant. For example, alkyl sulfates having an alkyl group of 10 to 18 carbon atoms, such as sodium cetyl sulfate, sodium lauryl sulfate, and sodium oleyl sulfate; polyoxyethylene alkyl ether sulfates having an average number of moles of ethylene oxide added of 2 to 4 and an alkyl group of 10 to 18 carbon atoms, such as sodium polyoxyethylene lauryl sulfate; alkyl sulfosuccinates having an alkyl group of 8 to 18 carbon atoms, such as sodium lauryl sulfosuccinate; natural surfactants, such as lecithin and glycerophospholipids; sphingophospholipids, such as sphingomyelin; and sucrose fatty acid esters having a fatty acid of 12 to 18 carbon atoms.

One of these surfactants or a combination of two or more of them can be used in the agent in the present invention. Preferable surfactants used in the formulation of the present invention are polyoxyethylene sorbitan fatty acid esters such as polysorbates 20, 40, 60, and 80, and among them, polysorbates 20 and 80 are particularly preferred. Polyoxyethylene polyoxypropylene glycols, as typified by poloxamer (e.g., Pluronic F-68 (registered trademark) etc.), are also preferred.

Examples of the buffer used in the present invention include phosphate buffer solution, citrate buffer solution, acetate buffer solution, malate buffer solution, tartrate buffer solution, succinate buffer solution, lactate buffer solution, potassium phosphate buffer solution, gluconate buffer solution, caprylate buffer solution, deoxycholate buffer solution, salicylate buffer solution, triethanolamine buffer solution, fumarate buffer solution, other organic acid-organic acid salt buffer solutions, carbonate buffer solution, Tris buffer solution, histidine buffer solution, and imidazole buffer solution.

A solution formulation may be prepared by dissolution in an aqueous buffer solution known in the preparation of solution formulations. The concentration of the buffer solution is generally 1 to 500 mM, preferably 5 to 100 mM, and more preferably 10 to 20 mM.

The agent in the present invention may comprise an additional ingredient. Examples of the additional ingredient include other low molecular weight polypeptides, other proteins such as serum albumin, gelatin and immunoglobulins, amino acids, sugars and carbohydrates such as polysaccharides and monosaccharides, and sugar alcohols.

In the present invention, the sugars and carbohydrates such as polysaccharides and monosaccharides include, for example, dextran, glucose, fructose, lactose, xylose, mannose, maltose, sucrose, trehalose, and raffinose.

In the present invention, the sugar alcohols include, for example, mannitol, sorbitol, and inositol.

When an aqueous solution for injection is prepared, for example, physiological saline, an isotonic solution containing glucose or another auxiliary substance (e.g., D-sorbitol, D-mannose, D-mannitol, sodium chloride, etc.), or the like can be used, optionally together with suitable solubilizers such as alcohols (e.g., ethanol etc.), polyalcohols (e.g., propylene glycol, PEG, etc.) and nonionic surfactants (e.g., polysorbate 80, HCO-50, etc.). If desired, diluents, solubilizers, pH adjusters, soothing agents, sulfur-containing reductants, and antioxidants may also be used.

If needed, the agent can be in the form of a microcapsule (made of hydroxymethylcellulose, gelatin, poly[methyl methacrylate], etc.) or a colloidal drug delivery system (liposomes, albumin microspheres, microemulsions, nanoparticles, nanocapsules, etc.) (see "Remington's Pharmaceutical Science 16th edition", Oslo Ed., 1980, etc.). Furthermore, methods for producing sustained release formulations are also known and can be applied to the present invention (Langer et al., J. Biomed. Mater. Res. 1981, 15: 167-277; Langer, Chem. Tech. 1982, 12: 98-105; U.S. Patent No. 3,773,919; European Patent Application Publication (EP) No. 58,481; Sidman et al., Biopolymers 1983, 22: 547-556; EP No. 133,988).

The carrier that is acceptable for use in the formulation may be a single carrier or a combination of carriers selected from the above-described materials as appropriate for the dosage form, but is not limited thereto.

When the Arid5A inhibitor of the present invention is used as a medicine for humans or other animals, the Arid5A inhibitor can be directly administered to a patient or formulated for administration to a patient according to methods known in the field of pharmaceutics. When the Arid5A inhibitor is formulated for administration, the above-described ingredients that are acceptable for use in the formulation may be incorporated.

The therapeutic agent for gastrointestinal cancer can be administered in the form of a pharmaceutical product and can be administered orally or parenterally; or systemically or locally. For example, intravenous injection such as infusion, intramuscular injection, intraperitoneal injection, subcutaneous injection, suppositories, enema, or enteric-coated oral preparations can be selected, and an appropriate administration method is selected for the age and symptoms of the patient. The effective dose is selected from the range of 0.001 mg to 100 mg per kilogram of body weight per administration. Alternatively, the dose can be selected from the range of 0.1 to 1,000 mg, preferably 0.1 to 50 mg per patient. Specifically, for example, a dosage of 0.1 mg to 40 mg, preferably 1 mg to 20 mg per kilogram of body weight is administered in one to several divided doses in a month (4 weeks) . For example, this dosage is administered in 2 divided doses/week, 1 dose/week, 1 dose/2 weeks, 1 dose/4 weeks, etc., by intravenous injection such as infusion, subcutaneous injection, etc. The dosing schedule can be adjusted while monitoring patient's condition and the trend of blood test values after administration. For example, the dosing interval can be increased from 2 times/week or once/week to once/2 weeks, once/3 weeks, or once/4 weeks.

In one embodiment, the Arid5A inhibitor inhibits epithelial-to-mesenchymal transition of cancer cells. The inhibition of epithelial-to-mesenchymal transition includes reduction of epithelial-to-mesenchymal transition. The epithelial-to-mesenchymal transition of cancer cells can be assessed, for example, by the method described in Example 4 below.

In one embodiment, the Arid5A inhibitor of the present invention inhibits cancer cell invasion. The inhibition of cancer cell invasion includes reduction of cancer cell invasion. The cancer cell invasion can be assessed, for example, by the method described in Example 4 below.

The therapeutic agent for gastrointestinal cancer may be used in combination with an additional cancer therapeutic agent or cancer therapy. The term "used in combination" herein means that the period of application of the therapeutic agent for gastrointestinal cancer of the present invention overlaps with the period of application of the additional cancer therapeutic agent or cancer therapy and does not always mean simultaneous administration or treatment. The additional cancer therapeutic agent or cancer therapy used in combination with the therapeutic agent for gastrointestinal cancer of the present invention is not particularly limited, and examples include chemotherapy (chemotherapeutic drugs), immunotherapy (immunotherapeutic drugs, immune checkpoint inhibitors, CAR-T therapeutic drugs, etc.), and hormone therapy (hormone therapy drugs).

### Screening method

The present invention provides a method for screening for a candidate substance useful for treating gastrointestinal cancer. The present inventors found that Arid5A is involved in immune evasion of pancreatic and colorectal cancer cells and demonstrated that Arid5A inhibition results in suppressed growth of pancreatic and colorectal cancers. Therefore, screening for substances capable of reducing Arid5A expression and/or function can provide candidate substances useful for treating gastrointestinal cancer.

The screening method of the present invention may comprise the steps of:
(1) examining whether a test substance affects Arid5A expression, and
(2) identifying the test substance as screen positive when the test substance is capable of reducing Arid5A expression based on comparison of cases with and without the test substance.

The screening method of the present invention may comprise the steps of:
(a) examining whether a test substance affects Arid5A function, and
(b) identifying the test substance as screen positive when the test substance is capable of reducing Arid5A function based on comparison of cases with and without the test substance.

In the screening of the present invention, the "test substance" is not particularly limited, and examples include single substances such as natural compounds, organic compounds, inorganic compounds, nucleic acid oligomers, proteins, and polypeptides; and compound libraries, nucleic acid oligomer libraries, polypeptide libraries, gene libraries, expression products of gene libraries, cell extracts, cell culture supernatants, fermentation microbial products, marine organism extracts, plant extracts, prokaryotic cell extracts, eukaryotic single cell extracts, and animal cell extracts. The test substances described above can be used after labeling if necessary. Examples of the labeling include radiolabeling and fluorescent labeling. In addition to the test substances described above, mixtures of these test substances are also used.

In a first embodiment of the screening method, the first is to bring Arid5A into contact with a test substance.

The Arid5A used in the screening method of the present invention may be a human Arid5A or a non-human Arid5A (e.g., from monkey, mouse, rat, guinea pig, pig, cow, yeast, insect, etc.). The amino acid sequence of the human Arid5A is as described above. The Arid5A used in the screening method of the present invention includes proteins that are functionally equivalent to known Arid5As. Such proteins include, but are not limited to, Arid5A mutants, Arid5A alleles, Arid5A variants, Arid5A homologs, partial peptides of Arid5A, and fusion proteins of Arid5A with another protein.

Examples of the Arid5A mutant include naturally occurring proteins that consists of a known amino acid sequence of Arid5A except for one or more amino acid substitutions, deletions, insertions, and/or additions and are functionally equivalent to the protein consisting of the known amino acid sequence. Other examples of the Arid5A mutant include proteins that are encoded by a naturally occurring DNA capable of hybridizing with a DNA consisting of a known nucleotide sequence encoding Arid5A under stringent conditions and are functionally equivalent to the protein consisting of the known amino acid sequence.

In the present invention, the number of the mutated amino acids is not particularly limited and is usually 30 or less, preferably 15 or less, and more preferably 5 or less (e.g., 3 or less) . For the mutated amino acid residue, it is desirable that the original amino acid is mutated to another amino acid in which the property of the amino acid side chain is conserved (conservative substitution). Examples of the property of the amino acid side chain include hydrophobic amino acids (A, I, L, M, F, P, W, Y, V), hydrophilic amino acids (R, D, N, C, E, Q, G, H, K, S, T), amino acids with an aliphatic side chain (G, A, V, L, I, P), amino acids with a hydroxyl-containing side chain (S, T, Y), amino acids with a sulfur-containing side chain (C, M), amino acids with a calboxyl- and amide-containing side chain (D, N, E, Q), amino acids with a base-containing side chain (R, K, H), and amino acids with an aromatic-containing side chain (H, F, Y, W). Note that the letters in parentheses represent one-letter codes for amino acids. It is already known that polypeptides having a modified amino acid sequence with one or more amino acid residue deletion, addition and/or substitution retain the same biological activities as those of the native polypeptides.

The term "functionally equivalent" herein means that a protein of interest has biological or biochemical functions equivalent to those of Arid5A. In one embodiment, Arid5A specifically binds to a stem-loop of IL-6 mRNA and antagonistically inhibits the action of Regnase-1, which specifically destroys IL-6 mRNA, thereby stabilizing IL-6 mRNA. In the present invention, the biological or biochemical functions of Arid5A include stabilization of IL-6 mRNA. In another embodiment, the biological or biochemical functions of Arid5A include stabilization of IDO1 mRNA. In yet another embodiment, the biological or biochemical functions of Arid5A include stabilization of CXCL3 mRNA, CCL2 mRNA, CCL5 mRNA, CCL7 mRNA, or CCL8 mRNA.

For preparation of a DNA encoding a "protein functionally equivalent" to a native protein of interest, methods well known to those skilled in the art are available and include hybridization or polymerase chain reaction (PCR) techniques. In other words, it is usual practice for those skilled in the art to isolate a DNA with a high homology to Arid5A by using a probe designed from the nucleotide sequence or a partial nucleotide sequence of Arid5A or using an oligonucleotide primer capable of specifically hybridizing to Arid5A.

In order to isolate such a DNA, hybridization is carried out preferably under stringent conditions. In the present invention, stringent hybridization conditions refer to conditions of 6 M urea, 0.4% SDS, and 0.5× SSC or other equivalently stringent conditions. More stringent conditions, e.g., 6 M urea, 0.4% SDS, and 0.1× SSC, are expected to enable isolation of a DNA with a higher homology. The thus isolated DNA is considered to provide a high homology at the amino acid level as compared to the amino acid sequence of the native protein of interest. High homology refers to at least 50% or more, preferably 70% or more, more preferably 90% or more (e.g. , 95%, 96%, 97%, 98%, 99% or more) sequence identity in the entire amino acid sequence. The amino acid or nucleotide sequence identity can be determined using the BLAST algorithm proposed by Karlin and Altschul (Proc. Natl. Acad. Sci. USA 87:2264-2268, 1990, Proc. Natl. Acad. Sci. USA 90:5873, 1993). Programs called BLASTN and BLASTX, which were developed based on the BLAST algorithm, are available (Altschul SF, et al: J Mol Biol 215:403, 1990). When BLASTN is used to analyze nucleotide sequences, the settings of the parameters are, for example, score = 100 and word length = 12. When BLASTX is used to analyze amino acid sequences, the settings of the parameters are, for example, score = 50 and word length = 3. When BLAST and Gapped BLAST programs are used, the default parameters of each program are used. The detailed procedures for these analysis methods are known.

The state of Arid5A used in the first embodiment of the screening method is not particularly limited, and for example, Arid5A may be purified, expressed in cells, or expressed in cell extracts.

The purification of Arid5A can be done by well-known methods . The cells expressing Arid5A include cells expressing endogenous Arid5A and cells expressing exogenous Arid5A. The cells expressing endogenous Arid5A can be, but are not limited to, cells from animal tissues and cultured cells. The cultured cells are not particularly limited, and for example, commercially available cells can be used. The organism species from which the cells expressing endogenous Arid5A are derived is not particularly limited, and examples include humans, monkeys, mice, rats, guinea pigs, pigs, cows, yeasts, and insects. The cells expressing exogenous Arid5A can be produced, for example, by introducing a vector containing a DNA encoding Arid5A into cells. The introduction of the vector into cells can be carried out by commonly used methods, such as calcium phosphate precipitation, electroporation, the lipofectamine method, and microinjection. Alternatively, the production of the cells containing exogenous Arid5A can be performed, for example, by chromosomally incorporating a DNA encoding Arid5A by a homologous recombination-based gene transfer method. The organism species from which the cells to be subjected to exogenous Arid5A introduction are derived is not limited to mammals and may be any organism species as long as cells from the organism species are compatible with well-established techniques for intracellular expression of exogenous proteins.

The cell extracts in which Arid5A is expressed can be, for example, cell extracts contained in an in vitro transcription-translation system to which a vector containing a DNA encoding Arid5A has been added. The in vitro transcription-translation system is not particularly limited, and commercially available in vitro transcription-translation kits can be used.

The "contact" in the first embodiment of the screening method is done in a manner suitable for the state of Arid5A. For example, when Arid5A is in a purified state, a test substance is added to the purified sample. When Arid5A is expressed in cells or cell extracts, a test substance is added to the culture medium of the cells or to the cell extracts, or a test substance is administered directly to experimental animals. When the test substance is a protein, for example, a vector containing a DNA encoding the protein is introduced into cells expressing Arid5A, or this vector is added to cell extracts in which Arid5A is expressed. Alternatively, a two-hybrid method using yeasts or animal cells can be used, for example.

In the first embodiment of the screening method, the next is to measure Arid5A expression and/or function. In one embodiment, examples of the Arid5A function include stabilization of IL-6 mRNA. Specifically, the Arid5A function can be indirectly measured by assessing the inhibition of IL-6 mRNA degradation in the presence of both Arid5A and the test substance. In the first embodiment, the next is to identify the test substance as screen positive when the test substance is capable of reducing or increasing Arid5A expression and/or function based on comparison of cases with and without contact with the test substance. When the test substance is administered to experimental animals, the spleen or other tissues are dissected from the treated animals for measuring Arid5A expression and/or function, IL-6 mRNA expression levels, etc. in specific cells such as macrophages. For the measurement of the expression level, amplification-based techniques such as PCR (polymerase chain reaction), RT-PCR (reverse transcription polymerase chain reaction), and real-time PCR, hybridization-based techniques, and/or other detection techniques can be selected as appropriate. The term "PCR" herein includes PCR and various types of PCR-derived amplification-based techniques for measuring DNA, RNA, etc. Since Arid5A is a protein that contributes to IL-6 mRNA stabilization, inhibition of intracellular Arid5A expression results in suppressed expression of IL-6 mRNA, but does not affect TNF-α mRNA expression as known previously. Therefore, by examining whether the test substance affects IL-6 mRNA expression without affecting TNF-α mRNA expression, it is possible to screen for substances that affect Arid5A expression, i.e., candidate substances useful for treating gastrointestinal cancer. The screen-positive test substances include substances capable of reducing Arid5A expression and/or function, which may result in therapeutic effect on gastrointestinal cancer. In a variation, IDO1 mRNA can be used similarly to IL-6 mRNA. In other variations, CXCL3 mRNA, CCL2 mRNA, CCL5 mRNA, CCL7 mRNA, and CCL8 mRNA can be used similarly to IL-6 mRNA.

In a second embodiment of the screening method, the first is to bring Arid5A into contact with a test substance, and the next is to measure the binding of Arid5A to the test substance. The measurement method is not particularly limited. The binding of Arid5A to the test substance can be measured using, for example, a label (e.g., a quantitatively measurable label such as a radiolabel or a fluorescent label) attached to the test substance bound to Arid5A. Alternatively, a labeling substance may be attached to Arid5A. The test substance or Arid5A can be immobilized on a resin or a chip to measure the binding. The functional change of Arid5A caused by the binding of the test substance to Arid5A can also be measured as an indicator.

In the second embodiment, the next is to identify the test substance as screen positive when the test substance is capable of binding to Arid5A. The screen-positive substances include substances capable of reducing Arid5A expression or function, which may result in therapeutic effect on gastrointestinal cancer.

In a third embodiment of the screening method, the first is to bring cells expressing Arid5A into contact with a test substance, and the next is to measure the Arid5A expression level. The measurement of the Arid5A expression level can be performed by methods known to those skilled in the art. For example, mRNA of the Arid5A gene is extracted according to the usual method, and the transcription level of the Arid5A gene is measured by northern hybridization or RT-PCR using the mRNA as a template. In addition, DNA array technology can also be used to measure the expression level of the Arid5A gene.

Alternatively, the translational level of the Arid5 gene may be measured. Specifically, the fractions containing Arid5A encoded by the Arid5 gene are collected according to the usual method, and electrophoresis such as SDS-PAGE is performed to measure the expression of Arid5A. Western blotting can also be used to measure the translation level of the Arid5 gene. Specifically, an antibody against Arid5A is used to measure the expression of Arid5A. Examples of the antibody against Arid5A include those described above.

In the third embodiment, the next is to identify the test substance as screen positive when the test substance is capable of reducing or increasing the expression level of Arid5A based on comparison of cases with and without contact with the test substance. The screen-positive substances include substances capable of reducing Arid5A expression, which may result in therapeutic effect on gastrointestinal cancer.

In a fourth embodiment of the screening method of the present invention, the first is to provide cells or cell extracts containing a DNA in which a reporter gene is functionally linked downstream of the promoter region of the DNA encoding Arid5A. In the fourth embodiment, "functionally linked" means that the reporter gene is linked to the promoter region of the Arid5A gene such that binding of a transcription factor to the promoter region of the Arid5A gene induces the expression of the reporter gene. Therefore, the term "functionally linked" includes cases in which the reporter gene is linked to another gene such that a fusion protein can be expressed as a gene product of both genes upon the binding of a transcription factor to the promoter region of the Arid5A gene.

The reporter gene is not particularly limited as long as its expression is detectable, and examples include reporter genes commonly used by those skilled in the art, such as a CAT gene, a lacZ gene, a luciferase gene, a β-glucuronidase gene (GUS), and a GFP gene. In addition, a DNA encoding Arid5A is also included in the scope of the reporter gene. The cells or cell extracts containing a DNA in which a reporter gene is functionally linked downstream of the promoter region of the DNA encoding Arid5A can be prepared in the same manner as described above.

In the fourth embodiment, the next is to bring the cells or cell extracts into contact with a test substance. Then, the expression level of the reporter gene in the cells or cell extracts is measured. The expression level of the reporter gene can be measured by methods known to those skilled in the art, from which a suitable one is selected for the type of the reporter gene used. For example, when the reporter gene is a CAT gene, the expression level of the reporter gene can be measured by quantifying the acetylation of chloramphenicol induced by the gene product. When the reporter gene is a lacZ gene, the expression level of the reporter gene can be measured by quantifying color emission from a dye compound catalyzed by the gene product. When the reporter gene is a luciferase gene, the expression level of the reporter gene can be measured by quantifying light emission from luciferin catalyzed by the gene product. When the reporter gene is a β-glucuronidase gene (GUS), the expression level of the reporter gene can be measured by quantifying light emission from glucuron (ICN) or color emission from 5-bromo-4-chloro-3-indolyl-β-glucuronide (X-Gluc) catalyzed by the gene product. When the Arid5A gene is used as the reporter gene, the expression level of the Arid5A gene can be measured in the same manner as described above.

In the fourth embodiment, the next is to identify the test substance as screen positive when the test substance is capable of reducing or increasing the expression level of the reporter gene based on comparison of cases with and without contact with the test substance. The screen-positive substances include substances capable of reducing Arid5A expression, which may result in therapeutic effect on gastrointestinal cancer.

In a fifth embodiment of the screening method, the first is to bring IL-6 mRNA into contact with a test substance, and the next is to measure the binding of IL-6 mRNA to the test substance. The measurement method is not particularly limited. The binding of IL-6 mRNA to the test substance can be measured using, for example, a label (e.g., a quantitatively measurable label such as a radiolabel or a fluorescent label) attached to the test substance bound to IL-6 mRNA. Alternatively, a labeling substance may be attached to IL-6 mRNA. The test substance or IL-6 mRNA can be immobilized on a resin or a chip to measure the binding. The activity change of IL-6 mRNA caused by the binding of the test substance to IL-6 mRNA can also be measured as an indicator. In a variation, IDO1 mRNA can be used similarly to IL-6 mRNA. In other variations, CXCL3 mRNA, CCL2 mRNA, CCL5 mRNA, CCL7 mRNA, and CCL8 mRNA can be used similarly to IL-6 mRNA.

In the fifth embodiment, the next is to identify the test substance as screen positive when the test substance is capable of binding to IL-6 mRNA. The screen-positive substances include substances capable of reducing Arid5A expression and/or function, which may result in therapeutic effect on gastrointestinal cancer. In a variation, IDO1 mRNA can be used similarly to IL-6 mRNA. In other variations, CXCL3 mRNA, CCL2 mRNA, CCL5 mRNA, CCL7 mRNA, and CCL8 mRNA can be used similarly to IL-6 mRNA.

In a sixth embodiment of the screening method, the first is to bring IL-6 mRNA into contact with a test substance and subsequently with Arid5A, and the next is to measure Arid5A function. The Arid5A function can be measured in the same manner as described above. In this embodiment, the next is to identify the test substance as screen positive when the test substance is capable of reducing or increasing Arid5A function based on comparison of cases with and without contact with the test substance. The screen-positive test substances include substances capable of reducing Arid5A function, which may result in therapeutic effect on gastrointestinal cancer. In a variation, IDO1 mRNA can be used similarly to IL-6 mRNA. In other variations, CXCL3 mRNA, CCL2 mRNA, CCL5 mRNA, CCL7 mRNA, and CCL8 mRNA can be used similarly to IL-6 mRNA.

### EXAMPLES

Hereinafter, the present invention will be described in detail by examples, but the present invention is not limited thereto.

### 1. Expression analysis of Arid5a protein in mouse embryonic fibroblasts (MEFs), B16/F10 cells, and KPC cells

KPC cells were received from Dr. Kodama from Kyoto University. B16/F10 cells were purchased from American Type Culture Collection (ATCC). MEFs were prepared in inventors' laboratory. The KPC cell is a cell prepared from KPC (LSL-KrasG 12D/+; LSL-Trp53R172H/+; Pdx-1-Cre) mice established as a model of human pancreatic ductal adenocarcinoma (PDAC).

A lysate of each cell type was prepared according to the usual method and subjected to SDS-PAGE. The samples were then transferred onto a PVDF membrane for western blot analysis. Anti-human Arid5a antibody (clone P18112, Thermo Fisher) was used as the primary antibody, and Amersham ECL anti-mouse IgG HRP conjugated (GE Healthcare) was used as the secondary antibody. Blots were developed using a chemiluminescence detection system (Chemi-Lumi One Ultra, Nacalai Tesque), and images were taken with an Image Quant LAS 500 (GE Healthcare).

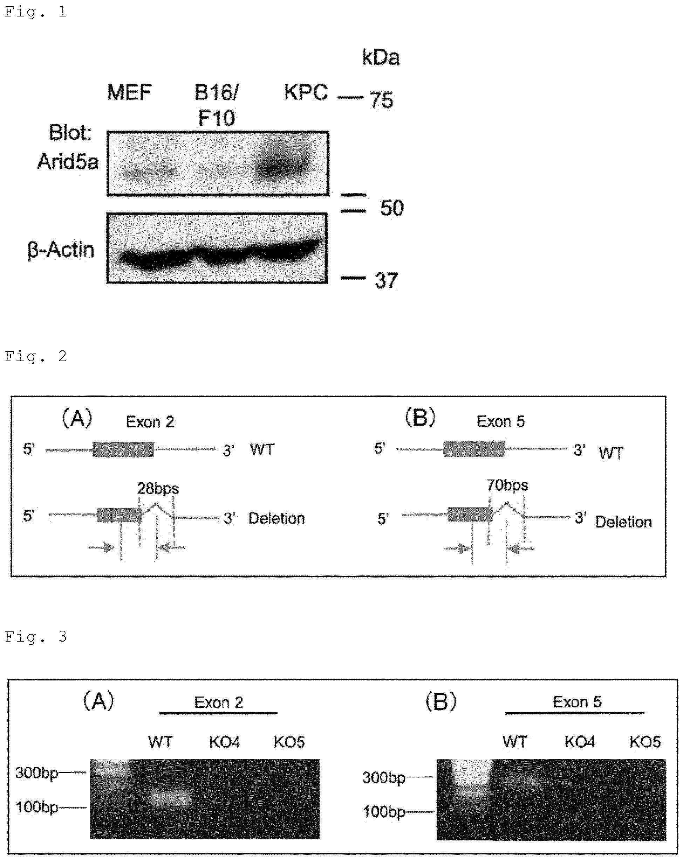

The results are shown in Fig. 1. KPC cells expressed a higher level of Arid5a as compared to MEFs and B16/F10 cells.

### 2. Generation of Arid5a-deficient KPC cells

The Arid5a gene in KPC cells was deleted using the CRISPR/Cas9 system (Santa Cruz) according to the manufacturer's instructions. For this purpose, a single guide RNA (sgRNA) designed to delete 28 base pairs around the 3' region of exon 2 of the Arid5a gene and an sgRNA designed to delete 70 base pairs around the 3' region of exon 5 of the Arid5a gene were used. The schematic views of the deletion regions of exon 2 and exon 5 of the Arid5a gene are shown in Fig. 2. Figs. 2A and 2B are schematic views of exon 2 and exon 5, respectively. The arrows at the bottoms indicate the positions of the primers for PCR.

The deletions of exon 2 and exon 5 of the Arid5a gene were verified by PCR. The results are shown in Fig. 3. Figs. 3A and 3B show the results for exon 2 and exon 5, respectively. Two Arid5a-deficient cell clones (KO4 and KO5) were obtained.

The Arid5a mRNA and protein levels in the obtained Arid5a-deficient cells were measured by qRT-PCR and western blot analysis, respectively, and compared with those in wild-type (WT) KPC cells that do not lack Arid5a.

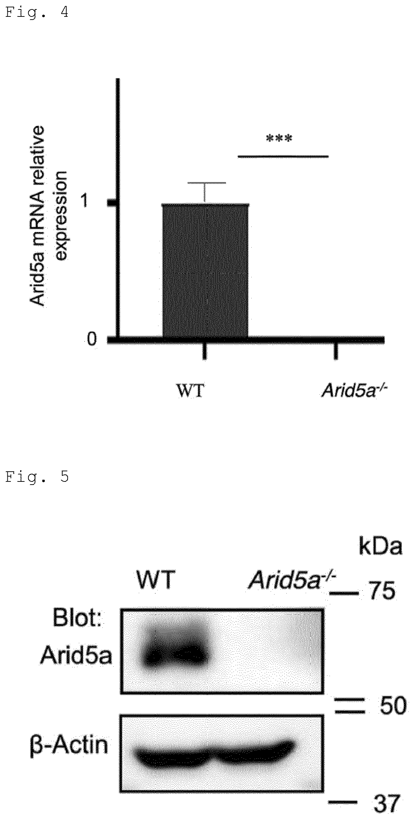

The results of Arid5a mRNA levels in the Arid5a-deficient KPC cell clone KO4 and wild-type (WT) KPC cells are shown in Fig. 4, and the results of Arid5a protein levels are shown in Fig. 5. In Arid5a-deficient KPC cells, Arid5a was not detected at the mRNA or protein level.

### 3. Measurement of the volume of Arid5a-deficient KPC cell tumors formed subcutaneously in mice

Five- to seven-week-old female BALB/c nude mice and five-to seven-week-old female C57BL/6 were used. 2 × 10⁵ cells each of wild-type (WT) KPC cells (hereafter referred to as "WT cells") and Arid5a-deficient KPC cells (hereafter referred to as "Arid5a-/- cells") were separately mixed with an equal volume of Matrigel and injected subcutaneously into the left and right flanks of the mice. The longest dimension (L) and perpendicular dimension (W) of the tumor were measured using a caliper on days 5, 7, 10, 14, 17, and 21. Tumor volume was calculated by the formula: L × W² × 0.5.

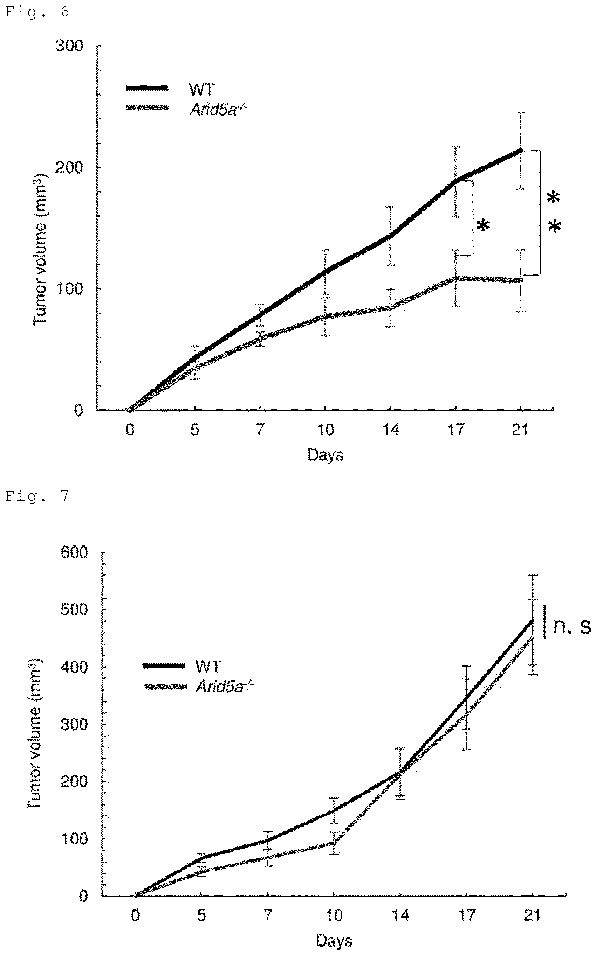

The results for C57BL/6 mice are shown in Fig. 6, and the results for BALB/c nude mice are shown in Fig. 7. Data are representative of three independent experiments and are presented as means ± SDs. Statistical analysis was performed using a Student's t-test. n.s. indicates no significant difference, * indicates P<0.05, and ** indicates P<0.01.

As shown in Fig. 6, when tumors were formed subcutaneously in C57BL/6 mice, the volume of Arid5a-/- tumors was significantly smaller than that of WT tumors after day 17. In contrast, when tumors were formed subcutaneously in BALB/c nude mice, Arid5a-/- and WT tumors showed a similar growth rate as shown in Fig. 7. In other words, the increase rate of the volume of Arid5a-/- tumors was suppressed in normal immunocompetent C57BL/6 mice, but not in immunodeficient mice (BALB/c nude mice) . These results indicate that Arid5a is involved in immune evasion of KPC cells. Therefore, Arid5a inhibition could result in suppression of pancreatic cancer growth and be effective in treating pancreatic cancer.

### 4. Investigation on involvement of Arid5a in epithelial-to-mesenchymal transition (EMT) of KPC cells

### (4-1) Morphological changes of KPC cells

WT and Arid5a-/- cells were cultured in the presence or absence of IL-6 (10 ng/mL) or TGFβ (5 ng/mL) for 48 hours, and cell morphology was microscopically analyzed. For cell culture, DMEM medium (Sigma) containing 10% FCS was used.

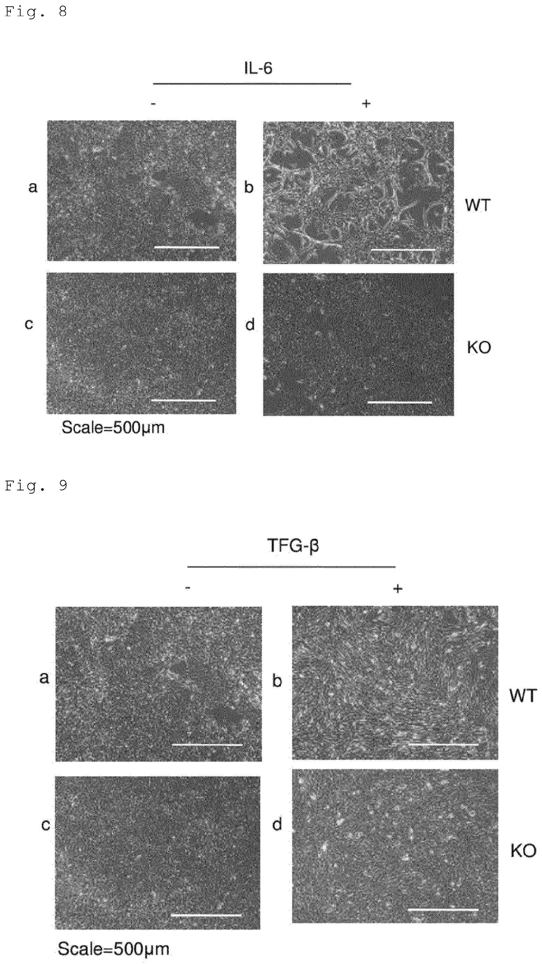

The results of IL-6 treatment are shown in Fig. 8. As is evident from Fig. 8b, the morphology of WT cells treated with IL-6 changed to a tubular structure. In contrast, Arid5a-/- cells did not show any morphological changes after IL-6 treatment (Fig. 8d). The results of TGFβ treatment are shown in Fig. 9. Arid5a-/- cells did not show any morphological changes after TGFβ treatment (Fig. 9d). These results indicate that Arid5a inhibition can prevent epithelial-to-mesenchymal transition of KPC cells.

### (4-2) E-cadherin expression

E-cadherin expression is known to be a hallmark of epithelial-to-mesenchymal transition (EMT). We tested E-cadherin expression in KPC cells (WT and Arid5a-/- cells).

WT and Arid5a-/- cells were cultured in the presence or absence of IL-6 (10 ng/mL) or TGFβ (5 ng/mL) for 48 hours, and E-cadherin expression was analyzed by western blot analysis.

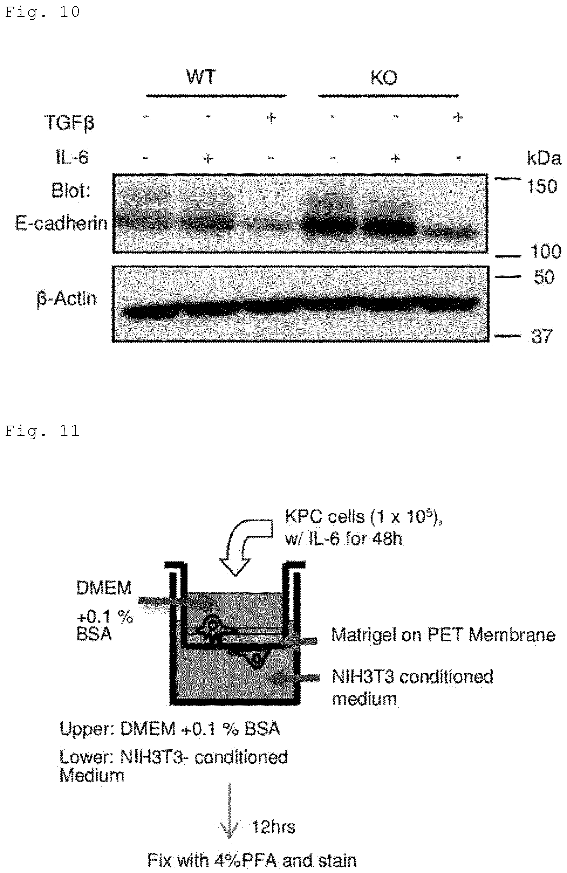

The results are shown in Fig. 10. In both WT and Arid5a-/- cells, there was no difference in E-cadherin expression between the presence and absence of IL-6. In contrast, E-cadherin expression was lower in the presence of TGFβ as compared to that in the absence of TGFβ in both WT and Arid5a-/- cells . However, in both conditions, E-cadherin expression was higher in Arid5a-/- cells than in WT cells, indicating that Arid5a may be involved in the maintenance of the mesenchymal phenotype.

### (4-3) IL-6-induced Matrigel invasion assay

Epithelial-to-mesenchymal transition is associated with cell invasiveness. We performed an IL-6-induced Matrigel invasion assay to determine whether Arid5a plays a role in the invasion activity of KPC cells.

Three types of cells were used for this assay: WT cells, Arid5a-/- cells, and Arid5a-/- cells transfected with an Arid5a expression vector. For each cell type, 1 × 10⁵ cells were incubated in the presence or absence of IL-6 (10 ng/mL) for 48 hours. The cells were then seeded into the upper wells with membranes in the Biocoat Matrigel chamber (BD Biosciences) in the absence of serum. The lower wells were filled with an NIH3T3-conditioned medium, in which NIH3T3 cells had been cultured in the absence of serum for 24 hours. After 12 hours of incubation, the cells invaded into Matrigel and migrated out onto the underside of the membranes. Each membrane was then fixed in 4% paraformaldehyde, and the number of cells onto the underside of the membrane was counted (see Fig. 11).

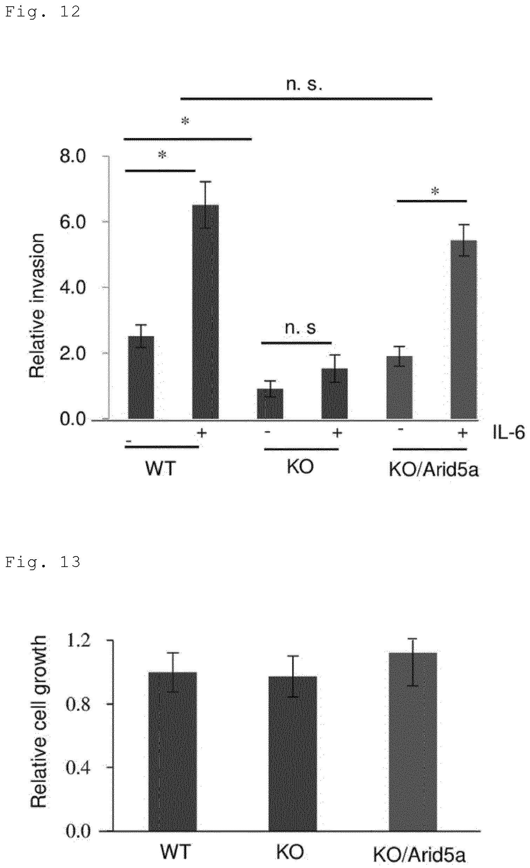

The results are shown in Fig. 12. Data are representative of three independent experiments and are presented as means ± SDs. Statistical analysis was performed using a Student's t-test. n.s. indicates no significant difference, * indicates P<0.05, and ** indicates P<0.01.

The IL-6-induced invasion activity of Arid5a-/- cells was significantly lower than that of WT cells. This low invasion activity of Arid5a-/- cells was rescued by Arid5a expression.

To exclude the possibility that the results of the invasion assay might be affected by cell growth, we monitored cell growth of WT cells, Arid5a-/- cells and Arid5a-expressing Arid5a-/- cells for relative comparison. The results are shown in Fig. 13. Data are representative of three independent experiments and are presented as means ± SDs. As is evident from Fig. 13, the three cell lines were comparably grown.

These results indicate that Arid5a is involved in the maintenance of the mesenchymal phenotype and in IL-6-induced invasiveness in pancreatic ductal adenocarcinoma (PDAC).

### 5. Investigation on involvement of Arid5a in IFN-γ-mediated Idol expression in tumors

### (5-1) RNA expression analysis

To identify the molecular mechanism of Arid5a-mediated immune evasion, we performed RNA sequencing of WT and Arid5a-/- cells.

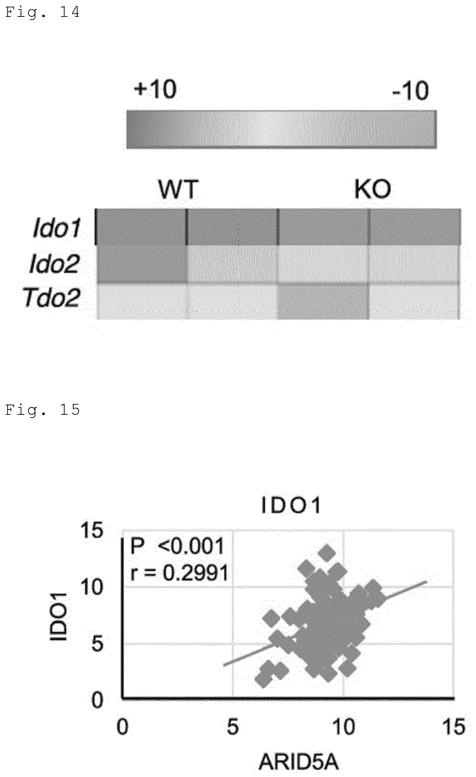

The results are shown in Fig. 14. The expression of Idol was significantly lower in Arid5a-/- cells, whereas the expressions of Ido2 and Tod2 were unaffected by Arid5a deletion.

### (5-2) The Cancer Genome Atlas (TCGA) dataset analysis

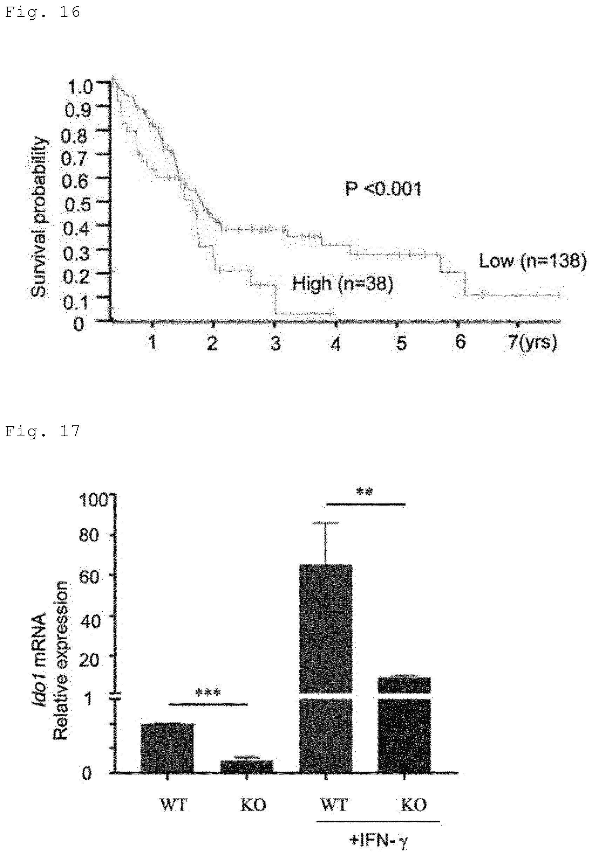

We found a statistically significant positive correlation between IDO1 expression and ARID5a expression in primary pancreatic ductal adenocarcinoma (PDAC) patients (Fig. 15). We also found that high expression of IDO1 mRNA in patients (top 27.5%; 38/138) statistically correlates with poor survival probability (Fig. 16).

### (5-3) Idol expression in Arid5a-/- cells

WT and Arid5a-/- cells were cultured in the presence or absence of IFN-γ (5 ng/mL) for 48 hours, and the expression level of Idol mRNA was measured by qRT-PCR. Data are representative of three independent experiments and are presented as means ± SDs.

In addition, WT and Arid5a-/- cells were cultured in the presence or absence of IFN-γ (5 ng/mL) for 0, 24, 48, and 72 hours, and Arid5a and Idol proteins were measured by western blot analysis.

The results for Idol mRNA are shown in Fig. 17. In the absence of IFN-γ, the expression of Idol mRNA in Arid5a-/- cells was substantially abolished.

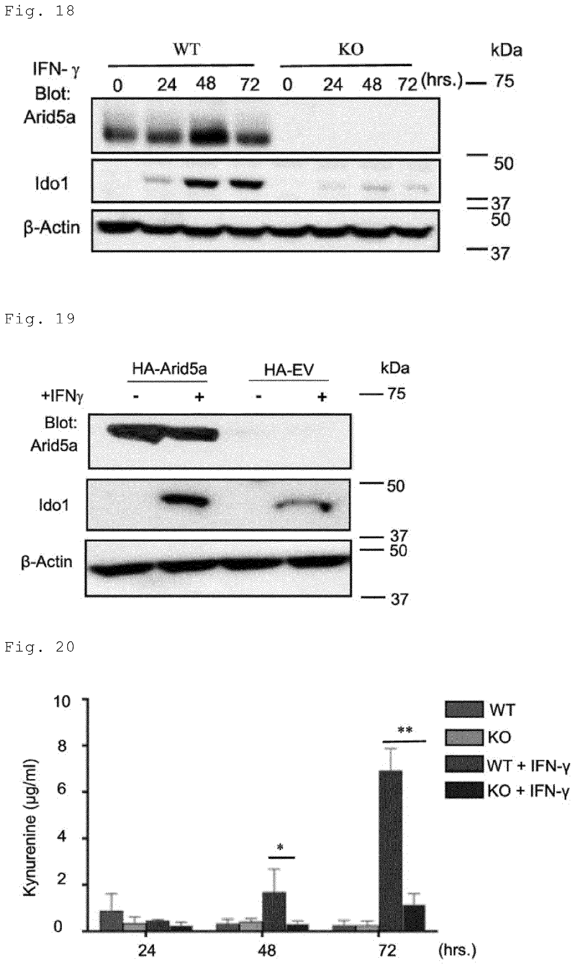

The results for Idol protein are shown in Fig. 18. In WT cells, Arid5a expression increased in 48 hours in response to IFN-γ. In addition, IFN-γ-induced Idol expression was observed at 24 hours and further increased at 48 hours. This indicates a possible correlation between Arid5a and Idol expressions. However, Idol expression in Arid5a-/- KPC cells was substantially lower even in the presence of IFN-γ stimulation.

### (5-4) Idol expression in HA-Arid5a-overexpressing Arid5a-/- cells

Arid5a-/- cells transfected with an HA-Arid5a expression vector and Arid5a-/- cells transfected with an empty vector (HA-EV) were cultured in the presence or absence of IFN-γ (5 ng/mL) for 48 hours, and Arid5a and Idol proteins were measured by western blot analysis. The results are shown in Fig. 19. The level of IFN-γ-induced Idol protein expression in HA-Arid5a-overexpressing Arid5a-/- cells was higher than that in empty vector (HA-EV)-transfected Arid5a-/- cells.

### (5-5) Kynurenine expression in Arid5a-/- cells

Idol has been reported to catabolize tryptophan to kynurenine, which activates AhR and promotes immune evasion through Treg differentiation. In this study, WT and Arid5a-/- cells were cultured in the presence or absence of IFN-γ (5 ng/mL) for 24, 48, and 72 hours, and the kynurenine level in the supernatant was measured by ELISA.

The results are shown in Fig. 20. Data are representative of three independent experiments and are presented as means ± SDs. Arid5a-/- cells consistently showed a lower level of kynurenine in the supernatant even when treated with IFN-γ. These results indicate that Arid5a plays an important role in immune evasion through the regulation of Idol-kynurenine axis.

### (5-6) 3'-UTR luciferase assay in Arid5a-/- cells

Arid5a has been identified as a post-transcriptional regulator and is known to stabilize its target mRNAs such as IL-6 and Stat3 mRNAs by binding to their 3'-UTR. To examine whether Arid5a would regulate Idol expression by stabilizing Idol mRNA, we performed a 3'-UTR luciferase assay using Arid5a-/- KPC cells.

Arid5a-/- cells cultured in a 24-well plate were transfected with a pGL3-luciferase plasmid encoding the Idol 3'-UTR region or a pGL3-luciferase control plasmid (Promega). All transfections were performed in combination with an Arid5a pcDNA3-Flag-overexpressing vector or an empty pcDNA3-Flag vector. A Renilla luciferase reporter plasmid (Promega) was transfected as an internal standard. After 24 hours, the cells were lysed with a passive lysis buffer. Luciferase activity was measured using a Dual Luciferase Reporter Assay System (Promega).

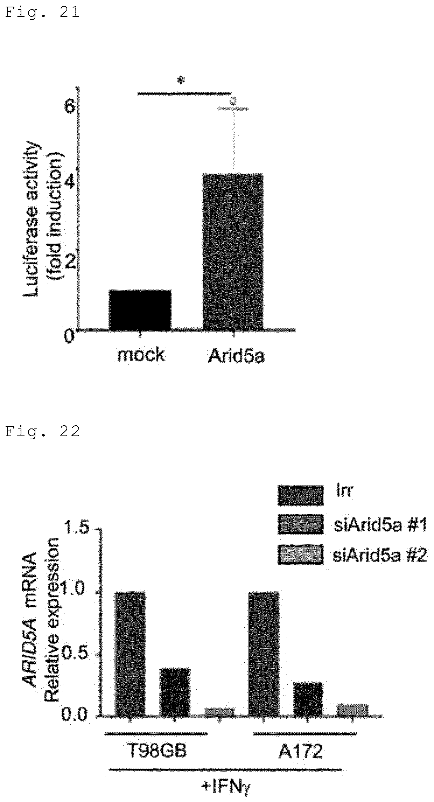

The results are shown in Fig. 21. Data are representative of three independent experiments and are presented as means ± SDs. "Mock" represents empty vector-transfected cells and "Arid5a" represents HA-Arid5a-overexpressing cells. Surprisingly, Arid5a overexpression resulted in stabilization of the Idol 3'-UTR.

### (5-7) Arid5a knockdown study

We knocked down ARID5A and IDO1 in the human glioblastoma cell lines A172 and T98G, which have been reported to express IDO1 in response to IFN-γ stimulation.

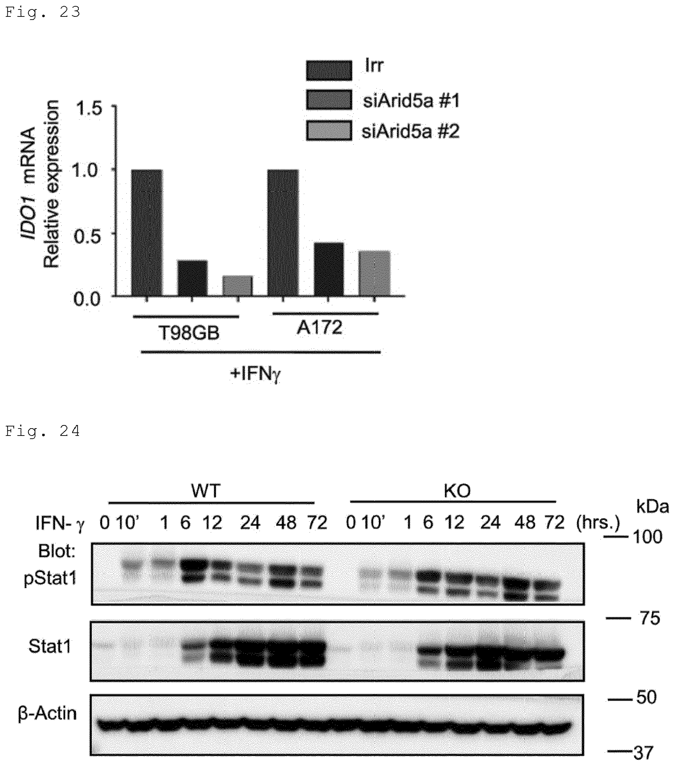

Fig. 22 shows the expression levels of Arid5a mRNA in each cell line transfected with Arid5a siRNAs. In both cell lines, Arid5a mRNA expression was reduced by Arid5a siRNA transfection, indicating that the siRNAs were functional. Fig. 23 shows the expression levels of Idol mRNA in each cell line transfected with Arid5a siRNAs. In both cell lines, Idol mRNA expression was reduced by Arid5a siRNA transfection. This is an interesting result showing that silencing of Arid5a suppresses Idol mRNA expression.

Taken together, these results indicate that Arid5a regulates Idol expression in mouse and human malignant tumor cells.

### 6. Investigation on involvement of Stat1 and Arid5a in IFN-γ-mediated regulation of Idol expression

### (6-1) Analysis for phosphorylated Stat1 (pStat1) in Arid5a-/- cells

IFN-γ activates JAK via an IFN receptor, and the activated JAK phosphorylates tyrosine at position 701 of Stat1. The phosphorylated Stat1 (pStat1) has been reported to activate Idol transcription by binding to its promoter. In this study, WT and Arid5a-/- cells were cultured in the presence or absence of IFN-γ (5 ng/mL) for 10 minutes, 1, 6, 12, 24, 48, and 72 hours, and Stat1 and pStat1 were measured by western blot analysis.

The results are shown in Fig. 24. In the absence of IFN-γ treatment (0 min), neither WT cells nor Arid5a-/- cells expressed Stat1. Treatment with IFN-γ for 6 hours or more resulted in the expression of Stat1 in both WT and Arid5a-/- cells. The level of pStat1 was comparable in WT and Arid5a-/- cells.

### (6-2) Expression of Arid5a and Idol in Stat1-silenced KPC cells

Stat1 shRNA was lentivirally introduced into KPC cells to generate Stat1-silenced KPC cells (#1 and #5). A scrambled shRNA was lentivirally introduced into KPC cells to generate control cells (Scr). Scr, #1 and #5 cells were treated with IFN-γ (5 ng/mL), and Arid5a and Idol were measured by western blot analysis.

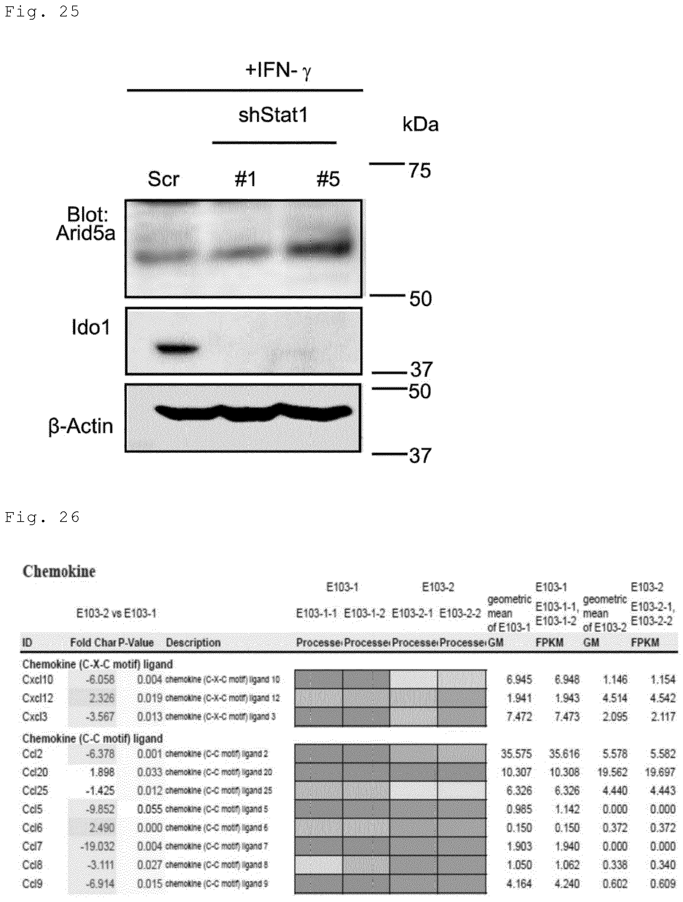

The results are shown in Fig. 25. Stat1 inhibition did not affect Arid5a expression, but completely abolished Idol expression. The results lead to a hypothesis that pStat1 activates the transcription initiation reaction of Idol and Arid5a stabilizes Idol mRNA post-transcriptionally.

### 7. Investigation on involvement of Arid5a in chemokine expression in tumors

### (7-1) RNA expression analysis

To investigate another possible molecular mechanism of Arid5a-mediated immune evasion, we examined changes in gene expression levels of various chemokines in WT and Arid5a-/- cells by RNA sequencing. Fragments per kilobase of exon per million mapped fragments (FPKM) values, which represent gene expression levels, were calculated using Cufflinks (http://cufflinks.cbcb.umd.edu) .

The results are shown in Fig. 26. In the figure, "E103-1" represents WT cells, and "E103-2" represents Arid5a-/- cells. We found that the expressions of chemokines such as CXCL10, CXCL3, CCL2, CCL5, CCL7, CCL8, and CCL9 were significantly lower in Arid5a-/- cells than in WT cells.

### (7-2) The Cancer Genome Atlas (TCGA) dataset analysis

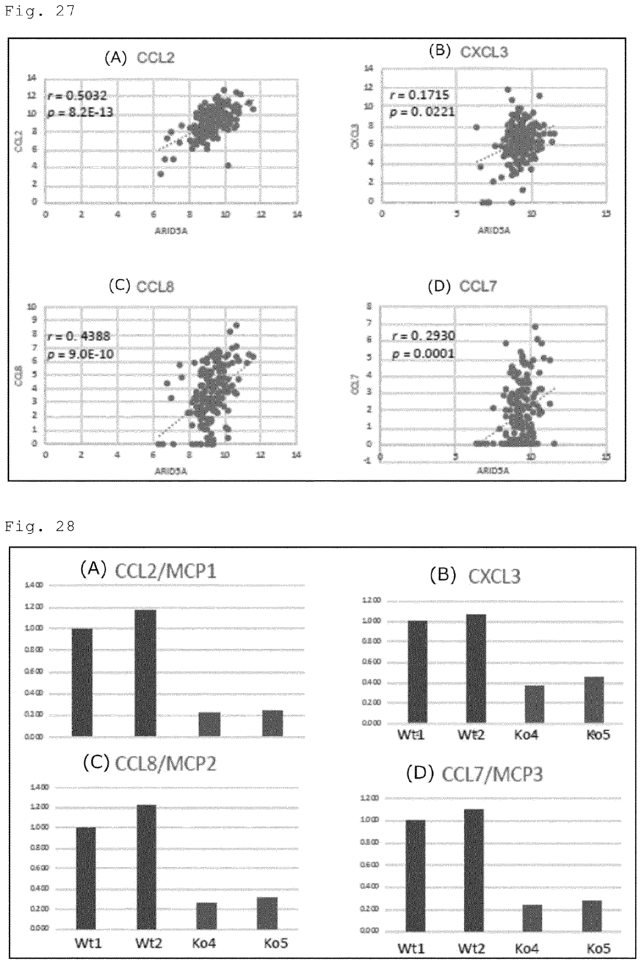

We found a statistically significant positive correlation between chemokine expressions (CXCL3, CCL2, CCL7, and CCL8 expressions) and ARID5a expression in primary pancreatic ductal adenocarcinoma (PDAC) patients (Fig. 27).

### (7-3) Chemokine expression in Arid5a-/- cells

WT and Arid5a-/- cells were cultured for 48 hours, and the mRNA expression levels of chemokines (CXCL3, CCL2, CCL7, and CCL8) were measured by qRT-PCR.

The results for each chemokine mRNA (CXCL3, CCL2, CCL7, or CCL8 mRNA) are shown in Fig. 28. The mRNA expressions of chemokines CXCL3, CCL2, CCL7, and CCL8 were lower in Arid5a-/- cells than in WT cells, indicating that Arid5a is involved in the regulation of the expression of these chemokines.

### (7-4) 3'-UTR luciferase assay in Arid5a-/- cells

We performed a 3' -UTR luciferase assay according to the same protocol as described in Example (5-6) to determine whether Arid5a would regulate chemokine expression by stabilizing chemokine mRNAs.

More specifically, Arid5a-/- cells cultured in a 24-well plate were transfected with a pGL3-luciferase plasmid encoding the chemokine CCL2 or CCL8 3'-UTR region or a pGL3-luciferase control plasmid (Promega). All transfections were performed in combination with an Arid5a pcDNA3-Flag-overexpressing vector or an empty pcDNA3-Flag vector. A Renilla luciferase reporter plasmid (Promega) was transfected as an internal standard. After 24 hours, the cells were lysed with a passive lysis buffer. Luciferase activity was measured using a Dual Luciferase Reporter Assay System (Promega).

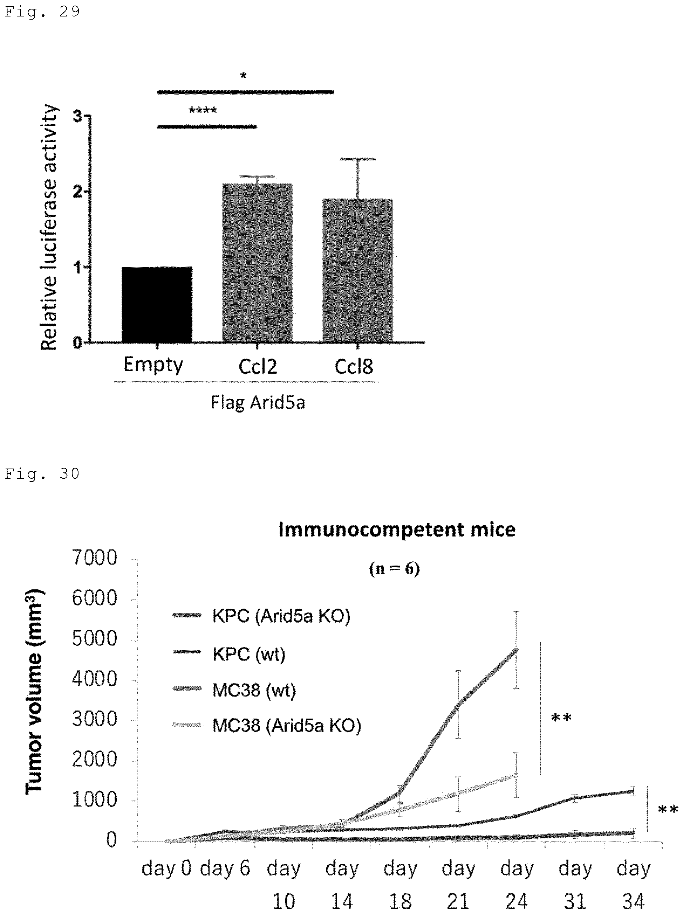

The results are shown in Fig. 29. Data are representative of three independent experiments and are presented as means ± SDs. "Empty" represents the results obtained using the control luciferase, "Ccl2" represents the results obtained using a luciferase additionally having the CCL2 3'-UTR region, and "Ccl8" represents the results obtained using a luciferase additionally having the CCL8 3'-UTR region. * indicates P<0.05, and **** indicates P<0.0001. The results of this experiment showed that Arid5a is capable of stabilizing the CCL2 and CCL8 3'-UTRs.

### 8. Investigation on involvement of Arid5a in colorectal cancer

### (8-1) Generation of Arid5a-deficient MC38 cells

The murine colorectal adenocarcinoma cell line MC38 was purchased from Kerafast, Inc. (Boston, USA). Arid5a-deficient MC38 cells were generated using the same technique as used in the generation of Arid5a-deficient KPC cells described in Example 2.

### (8-2) Measurement of the volume of Arid5a-deficient MC38 cell tumors formed subcutaneously in mice

Five- to seven-week-old female BALB/c nude mice and five-to seven-week-old female C57BL/6 were used. 2 × 10⁵ cells each of wild-type MC38 cells (hereafter referred to as "MC38_WT cells") and Arid5a-deficient MC38 cells (hereafter referred to as "MC38_Arid5a-/- cells") were separately mixed with an equal volume of Matrigel and injected subcutaneously into the left and right flanks of the mice. The longest dimension (L) and perpendicular dimension (W) of the tumor were measured using a caliper on days 6, 10, 14, 18, 21, and 24. Tumor volume was calculated by the formula: L × W² × 0.5.

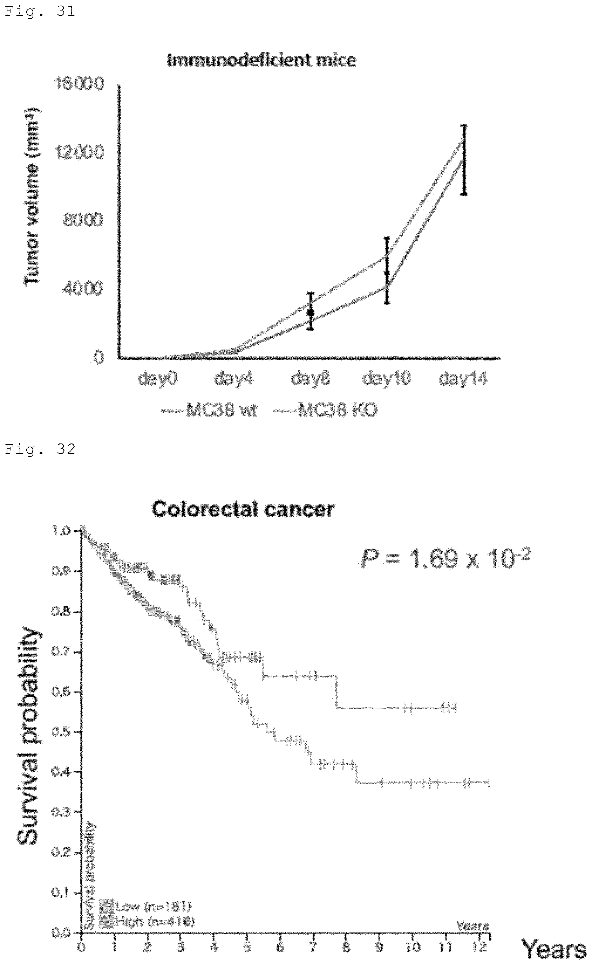

The results for C57BL/6 mice are shown in Fig. 30, and the results for BALB/c nude mice are shown in Fig. 31. Data are representative of three independent experiments and are presented as means ± SDs. Statistical analysis was performed using a Student's t-test. ** indicates P<0.01.

As shown in Fig. 30, when tumors were formed subcutaneously in C57BL/6 mice, the volume of MC38_Arid5a-/- tumors was significantly smaller than that of MC38_WT tumors. In contrast, when tumors were formed subcutaneously in BALB/c nude mice, MC38_Arid5a-/- and MC38_WT tumors showed a similar growth rate as shown in Fig. 31. In other words, the increase rate of the volume of MC38_Arid5a-/- tumors was suppressed in normal immunocompetent C57BL/6 mice, but not in immunodeficient mice (BALB/c nude mice). These results indicate that Arid5a is involved in immune evasion of MC38 cells. Therefore, Arid5a is involved in growth suppression of colorectal cancer as well as pancreatic cancer, and Arid5a inhibition could be effective in treating colorectal cancer as well.

### (8-3) The Cancer Genome Atlas (TCGA) dataset analysis

We found that colorectal cancer patients with high expression of Arid5a mRNA had a poorer survival probability than colorectal cancer patients with low expression of Arid5a mRNA, and Arid5a expression in colorectal cancer patients statistically correlates with survival probability (Fig. 32) .

The present invention is not limited to the particular embodiments and examples described above, and various modifications can be made within the scope of the appended claims. Other embodiments provided by suitably combining technical means disclosed in separate embodiments of the present invention are also within the technical scope of the present invention. All the academic publications and patent literature cited in the description are incorporated herein by reference.

## Claims

1. A therapeutic agent for gastrointestinal cancer, comprising an Arid5A inhibitor as an active ingredient.

2. The therapeutic agent according to claim 1, wherein the Arid5A inhibitor comprises a substance capable of reducing Arid5A expression or function.

3. The therapeutic agent according to claim 1 or 2, wherein the Arid5A inhibitor is a nucleic acid oligomer or a polypeptide.

4. The therapeutic agent according to claim 3, wherein the nucleic acid oligomer is selected from the group consisting of an siRNA, an shRNA, an antisense nucleic acid, a decoy nucleic acid, a nucleic acid aptamer, and a ribozyme.

5. The therapeutic agent according to claim 3, wherein the polypeptide is a cyclic polypeptide.

6. The therapeutic agent according to any one of claims 1 to 5, wherein the Arid5A inhibitor is capable of inhibiting epithelial-to-mesenchymal transition of cancer cells.

7. The therapeutic agent according to any one of claims 1 to 6, wherein the Arid5A inhibitor is capable of inhibiting cancer cell invasion.

8. The therapeutic agent according to any one of claims 1 to 7, wherein the gastrointestinal cancer is pancreatic cancer or colorectal cancer.

9. A method for screening for a candidate substance useful for treating gastrointestinal cancer, comprising the steps of:
(1) examining whether a test substance affects Arid5A expression, and
(2) identifying the test substance as screen positive when the test substance is capable of reducing Arid5A expression based on comparison of cases with and without the test substance.

10. A method for screening for a candidate substance useful for treating gastrointestinal cancer, comprising the steps of:
(a) examining whether a test substance affects Arid5A function, and
(b) identifying the test substance as screen positive when the test substance is capable of reducing Arid5A function based on comparison of cases with and without the test substance.

11. The screening method according to claim 10, wherein the Arid5A function is stabilization of any one mRNA selected from the group consisting of IL-6 mRNA, IDOl mRNA, CXCL3 mRNA, CCL2 mRNA, CCL5 mRNA, CCL7 mRNA, and CCL8 mRNA.

12. The screening method according to any one of claims 9 to 11, wherein the gastrointestinal cancer is pancreatic cancer or colorectal cancer.
